# EUROPEAN PATENT APPLICATION

(11) **EP 4 803 108 A1**
(43) Date of publication of application: **09.09.2026**
(21) Application number: 24885961.3
(22) Date of filing: 29.07.2024
(51) Int. Cl.: A61L 24/00, A61L 24/10, A61L 27/36, A61L 27/52, A61L 27/22

(54) **EXTRACELLULAR MATRIX-BASED ADHESIVE COMPOSITION**

(30) Priority: 30.10.2023 KR 20230146765
(71) Applicant: BioBricks Co., Ltd, Pohang-si, Gyeongsangbuk-do 37673 (KR)
(72) Inventor: JANG, Jin Ah, Pohang-si Gyeongsangbuk-do 37673 (KR); PARK, Juyoung, Dalseong-gun Daegu 42922 (KR); CHAE, Younhee, Gyeongju-si Gyeongsangbuk-do 38033 (KR); LEE, Yeonju, Daegu 41186 (KR)
(74) Representative: Gilani, Anwar
(86) International application number: PCT/KR2024/010993
(87) International publication number: WO 2025/095281

(57) **Abstract**

The present invention relates to an extracellular matrix-based adhesive composition, particularly characterized by comprising an extracellular matrix-containing hydrogel, methacryloyl-substituted gelatin, and a gelatin cross-linking agent, thereby providing a bioadhesive with excellent bioadhesion and elasticity, as well as a method for manufacturing the same and its uses.

## Description

### [Technical Fields]

The teachings in accordance with exemplary and non-limiting embodiments of the present invention relate generally to an extracellular matrix-based adhesive composition, particularly a bioadhesive based on extracellular matrix having excellent bioadhesive properties and elasticity, a method for manufacturing the same, and uses thereof.

This patent was developed with support from the Ministry of SMEs and Startups (MSS, Republic of Korea) under the Technology Development Project (S3318933).

### [Background Arts]

Biological adhesives are a general term for adhesive materials used to bond biological tissues such as skin, blood vessels, intestines, and bones, or to bond artificial materials to biological tissues. They primarily utilize substances that exhibit adhesive properties when applied to various biological samples, such as cells and proteins from living organisms. Biological adhesives have a wide range of clinical applications, including tissue adhesives, haemostats, tissue engineering scaffolds, drug delivery hydrogels, tissue fillers, and wound healing. Biological adhesives require strong adhesive and cross-linking capabilities, must maintain their functionality over extended periods within the body, and must adhere instantly in the presence of bodily fluids without generating heat or harmful substances, while also avoiding immune reactions.

Currently, commercially available or practical bioadhesives include cyanoacrylate instant adhesives, fibrin glue, gelatin glue, and polyurethane-based adhesives. However, bioadhesives made from synthetic polymers exhibit very weak bonding strength in the aqueous environment of the body. Additionally, cyanoacrylate-based, gelatin-based, and polyurethane-based bioadhesives have been reported to have biological toxicity and to cause adverse effects such as immune reactions in the human body, which are major limitations. Additionally, fibrin-based bioadhesives currently used in actual patients have few side effects but exhibit very low adhesive ability, limiting their practical application. Furthermore, most bioadhesives currently commercialized or in practical use have low biological activity, which can hinder self-healing at the injury site and impair tissue regeneration. To overcome these issues, the development of an ideal bioadhesive with strong adhesive and cross-linking capabilities, minimal adverse effects in the body, and the ability to promote tissue regeneration is necessary.

Meanwhile, the cornea is a transparent tissue located on the front surface of the eye, devoid of blood vessels, serving as the outermost layer of the eye's front portion and protecting it from external stimuli. As the first structure through which light enters the eye, it plays a crucial role in light refraction and transmission. However, the cornea is constantly exposed to the external environment, making it susceptible to injury and various diseases. According to the World Health Organization, approximately 285 million people worldwide suffer from visual impairments primarily caused by corneal diseases, including conditions such as dry eye syndrome, Sjögren's syndrome, and bacterial keratitis, as well as complications arising from these conditions. Chronic corneal diseases can cause corneal opacity, necessitating corneal transplantation, with over 1.5 million new cases of corneal blindness reported annually. However, due to a shortage of donor tissue and high surgical costs, less than 5% of patients receive transplant surgery. Corneal damage and infection can cause corneal scarring and thinning of the corneal tissue, which may lead to vision loss.

In cases of severe corneal diseases or trauma resulting in corneal damage, corneal transplantation is essential. However, globally, there is an absolute shortage of corneal donors compared to the number of patients awaiting transplantation, with the average waiting time for transplantation reaching approximately eight years. Therefore, various approaches are currently being employed, such as using tissue adhesives like cyanoacrylate adhesive and fibrin glue for conservative treatment, or transplanting amniotic membrane to protect wounds and reduce inflammation. However, in cases of severe corneal damage, there are no therapeutic agents worldwide that can induce healing and regeneration of the corneal epithelial tissue or stroma. To overcome these limitations, there is an urgent need for the development of biocompatible materials with high transparency, easy biocompatibility, and the ability to regenerate and repair tissues. In particular, biomaterials with high adhesion that can effectively adhere to tissues in humid environments such as the ocular surface are emerging as a new technology that can enhance the convenience of medical procedures in clinical settings.

In connection with this, the inventors have disclosed a composition in the form of an adhesive comprising a hydrogel containing extracellular matrix and a gelatin cross-linking agent, as disclosed in Korean Patent Application No. 10-2023-0050638 (Invention Title: Extracellular Matrix-Based Biocompatible Adhesive). wherein the extracellular matrix-containing hydrogel is characterized by being gelatinized. However, the aforementioned bioadhesive also has the drawbacks of relatively low adhesion strength and insufficient elasticity in response to compressive stress.

Therefore, there is an ongoing need for the development of a bioadhesive that possesses excellent adhesive strength while exhibiting elasticity similar to that of biological corneal tissue in response to compressive stimuli, thereby making it suitable for use in corneal tissue repair.

### [Related Prior Technical Documents]

### [Patent Documents]

Korean Patent Application No. 10-2023-0050638 (April 17, 2023)

### [Summary of the Invention]

### [Technical Subject]

The present invention is intended to solve the above-mentioned problems and provides an adhesive composition having excellent viscosity and mechanical strength.

Additionally, the present invention aims to provide an adhesive composition that not only exhibits excellent bioadhesive properties but also possesses elasticity in response to compressive stress, similar to that of biological corneal tissue.

Furthermore, the present invention is intended to provide a useful application as an adhesive biomaterial for the healing and/or regeneration of corneal defects and/or damage, such as dry eye syndrome or corneal ulcers.

### [Technical Solution]

The extracellular matrix-based adhesive composition according to the present invention for achieving the above-mentioned purpose may comprise an extracellular matrix-containing hydrogel, a gelatin crosslinking agent, and a methacryloyl-substituted gelatin.

Here, the methacryloyl-substituted gelatin may be included at a concentration of 5 to 15% (w/v) as GelMA (gelatin-methacryloyl).

Additionally, the methacryloyl-substituted gelatin may be included at a concentration of 5 to 10% (w/v) as GelMA.

Furthermore, the methacryloyl-substituted gelatin may be included at a concentration of 5% (w/v) as GelMA.

Additionally, the extracellular matrix-containing hydrogel may be a gelatinized form of extracellular matrix derived from corneal stroma.

Furthermore, the gelatin crosslinking agent may be a photopolymerizable initiator comprising a combination of ruthenium and sulphur.

Additionally, the extracellular matrix-based adhesive composition according to the present invention may be for corneal tissue repair.

Further details of other embodiments are included in the detailed description and drawings.

### [Advantageous Effects]

The present invention is characterized by containing a gelatin hardener and methacryloyl-substituted gelatin in an extracellular matrix-containing hydrogel, thereby providing an adhesive composition with excellent viscosity and mechanical strength.

Additionally, the present invention enables the provision of an adhesive composition that not only exhibits excellent bioadhesive properties but also possesses elasticity similar to that of biological corneal tissue in response to compressive stress by including the methacryloyl-substituted gelatin at a specific ratio.

The present invention is suitable for use in corneal tissue repair, thereby providing a useful application as an adhesive biomaterial for the healing and/or regeneration of corneal defects and/or damage, such as dry eye syndrome or corneal ulcers.

### [Brief Description of Drawings]

FIG. 1 is a schematic diagram showing the synthesis process of D-tyrosine activated by visible light according to an exemplary embodiment of the present invention.
FIG. 2 is a schematic diagram showing the synthesis process of dopamine from oxidized tyrosine according to an exemplary embodiment of the present invention.
FIG. 3 shows the results of protein amino acid analysis in Co-dECM hydrogel according to an exemplary embodiment of the present invention.
FIG. 4 shows the results of measuring the viscosity of an extracellular matrix-based adhesive composition according to an exemplary embodiment of the present invention, depending on the GelMA addition ratio.
FIG. 5 shows the results of analyzing the gelation kinetics based on the visible light irradiation time for each GelMA addition ratio in an extracellular matrix-based adhesive composition according to an exemplary embodiment of the present invention.
FIG. 6 shows the results of analyzing the mechanical strength (storage modulus) of the gel induced by photocross-linking agent in an extracellular matrix-based adhesive composition according to an exemplary embodiment of the present invention, depending on the GelMA addition ratio.
FIG. 7 shows the results of analyzing the lap shear strength in an extracellular matrix-based adhesive composition according to an exemplary embodiment of the present invention, depending on the GelMA addition ratio.
FIG. 8 shows the results of analyzing the elasticity in the extracellular matrix-based adhesive composition according to an exemplary embodiment of the present invention, depending on the GelMA addition ratio.

### [Best Mode]

The present invention is capable of various modifications and may have various embodiments. Therefore, specific embodiments are illustrated in the drawings and described in detail in the detailed description. However, this is not intended to limit the present invention to specific embodiments, and all modifications, equivalents, and substitutions included within the scope of the present invention's ideas and technical scope should be understood as included. In describing the present invention, detailed descriptions of related prior art that are deemed to obscure the essence of the present invention are omitted.

The terms used in this application are used solely to describe specific embodiments and are not intended to limit the scope of the present invention. Unless the context clearly indicates otherwise, singular expressions include plural expressions. In this application, terms such as 'include' or 'have' are intended to designate the presence of the features, numbers, steps, operations, components, parts, or combinations thereof described in the specification, and should not be understood as precluding the possibility of the presence or addition of one or more other features, numbers, steps, operations, components, parts, or combinations thereof.

Terms such as 'first,' 'second,' etc., may be used to describe various components, but such components are not limited to those described by such terms. Such terms are used solely for the purpose of distinguishing one component from another.

The extracellular matrix-based adhesive composition according to the present invention may comprise an extracellular matrix-containing hydrogel, a gelatin crosslinking agent, and a methacryloyl-substituted gelatin.

The extracellular matrix-containing hydrogel may be a hydrogel derived from or based on extracellular matrix. The term 'extracellular matrix (ECM)' used in the present invention refers to the extracellular portion of animal tissues that typically provides structural support to animal cells while performing various other important functions. The extracellular matrix is a characteristic feature defining connective tissue in animals and is composed of various forms of proteins, including collagen and glycosaminoglycans (GAGs). Such extracellular matrix may be derived from tissues of animals such as pigs or cows and can be extracted from various organs.

In the present invention, the extracellular matrix is preferably a decellularized extracellular matrix when considering applications such as in vivo applications. Decellularization is a technique that removes all foreign cells capable of inducing an immune response from biological tissues and separates the extracellular matrix. The present invention has developed a biomaterial for adhesive tissue repair using decellularized extracellular matrix (dECM) as the main component. In particular, decellularized extracellular matrix derived from corneal tissue contains numerous proteins essential for maintaining the differentiation, activity, and homeostasis of corneal tissue cells, thereby promoting the regeneration of corneal epithelium and stroma. Additionally, it can preserve the high transparency and mechanical properties characteristic of corneal tissue. The above-mentioned decellularized extracellular matrix has the effect of minimizing immune responses in allografts or xenografts by removing cells that can act as antigens that induce immune responses. Depending on the type of tissue, the number and type of cells, and the physical characteristics of the tissue itself, decellularization is achieved using various chemical substances such as acids, bases, storage solutions, buffers, and detergents. Additionally, the aforementioned extracellular matrix can be used as is after undergoing only the decellularization process to maintain the tissue's original structure. However, it can also be processed through freeze-drying and grinding, dissolved in an acidic solution, and then neutralized to form a hydrogel for use. Furthermore, considering the intended applications, decellularized extracellular matrix derived from the cornea is more preferable. The aforementioned corneal-derived decellularized extracellular matrix is derived from corneal stroma tissue and contains proteins that help cells attach and proteins that aid in the expression of cell growth and function, in addition to the physical structure surrounding the cells. The corneal-derived decellularized extracellular matrix preferably includes collagen fibers from which telopeptides have been removed.

In the present invention, the extracellular matrix-containing hydrogel may be gelatinized. The extracellular matrix-containing hydrogel may preferably be gelatinized by thermal denaturation of collagen, which is a constituent component of the extracellular matrix. The term 'gelatinized' used in the present invention refers to a state in which the extracellular matrix-containing hydrogel is denatured by heat and exhibits rheological properties identical or similar to those of gelatin. Gelatin is a type of derivative protein obtained by treating collagen with hot water. It swells in cold water but dissolves in hot water, forming a sol with flowability. The collagen, which is a major component of the extracellular matrix, denatures and dissolves when heated with water, releasing colloidal particles that convert into gelatin. Since collagen, a major component of the extracellular matrix, exists in a solidified state at body temperature (approximately 37°C) where biological adhesives are applied, it is difficult to evenly and easily apply a hydrated gel containing non-gelatinized extracellular matrix to the affected area, resulting in inconvenience in use. Additionally, even when a gelatin-hardening agent is added, non-gelatinized extracellular matrix-containing hydrogels do not harden easily, and their adhesion significantly decreases when applied to the lesion site.

Additionally, the extracellular matrix-containing hydrogel may be a gelatinized form of extracellular matrix derived from corneal stroma. The adhesive tissue repair biomaterial composition according to the present invention, when based on extracellular matrix derived from corneal tissue, includes not only collagen that constitutes corneal tissue but also naturally derived ECMs related to corneal tissue, such as those involved in eye development, wound healing, and tissue reconstruction, particularly including major ECMs such as keratans, lumicans, and decorins, which are associated with maintaining corneal homeostasis, thereby demonstrating excellent efficacy in regenerating damaged corneal tissue into clear and transparent original corneal tissue.

Additionally, in the extracellular matrix-based adhesive composition according to the present invention, the content of the extracellular matrix-containing hydrogel is not significantly limited, and considering factors such as gel formation ability, smooth thermal denaturation, uniform mixing with gelatin crosslinkers, or the convenience of use or adhesion of the bioadhesive, it is possible to be 1-5% (w/v), 2-4% (w/v) is preferable, and 1.5-3% (w/v) is even more preferable.

The adhesive composition according to the present invention, which includes a hydrogel containing extracellular matrix, not only has rheological properties identical or similar to those of gelatin but also has flowability at temperatures of 30°C or higher, enabling it to be evenly and easily applied to damaged lesions on the corneal surface, and can be rapidly cross-linked using visible light.

The gelatin hardening agent is a material that cures gelatin in the adhesive composition according to the present invention.

The term 'gelatin cross-linking agent (cross-linker, hardener, hardening agent may be cross-used in the specification)' used in this invention refers to a substance or combination of substances that cross-links gelatin through simple addition, heat treatment, or light irradiation, thereby converting it into a solid state. Examples of such gelatin cross-linking agents include inorganic compounds containing divalent metal ions, such as salts, and organic compounds such as aldehydes and quinones. Generally, light initiators used as gelatin hardening agents include Lithium phenyl-2,4,6-trimethylbenzoylphosphinate (LAP) and Irgacure 2959. Specific examples of gelatin hardening agents in this invention include: a combination of ruthenium (Ru) and sodium persulfate (SPS) that induces gelatin hardening upon exposure to visible light (especially blue light); riboflavin, which induces gelatin curing upon exposure to ultraviolet light; or EDC/NHS [(1-ethyl-3-(3-dimethylaminopropyl)carbodiimide hydrochloride/N-hydroxysuccinimide] , grape seed extract, dialdehyde starch, glutaraldehyde, etc.

In the extracellular matrix-based bioadhesive according to an embodiment of the present invention, the gelatin hardening agent may be a photoinitiator comprising a combination of ruthenium and sulphur, considering biocompatibility, ease of use, and adhesive properties. For example, the combination of ruthenium and sodium peroxymonosulphate or riboflavin may be selected, and more preferably, the combination of ruthenium and sodium peroxymonosulphate may be selected. The ruthenium and sodium peroxymonosulfate are known to oxidize aromatic residues containing tyrosine when exposed to visible light (especially blue light), and the oxidized aromatic residues are converted into free radical forms, which then form covalent bonds, such as di-tyrosine covalent bonds, thereby inducing a cross-linking reaction.

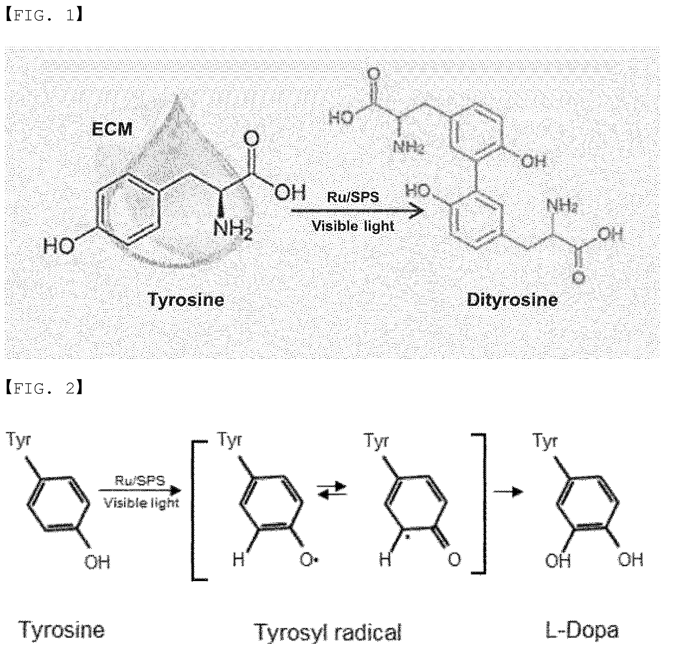

FIG. 1 is a schematic diagram illustrating the synthesis process of di-tyrosine activated by visible light according to an exemplary embodiment of the present invention. When a photoreceptor, such as ruthenium and sodium peroxymonosulphate, is incorporated into a bio-derived extracellular matrix hydrogel, and visible light is irradiated, Ru2+ is photodecomposed to Ru3+ in the presence of an electron acceptor, SPS. The presence of Ru³⁺ subsequently oxidizes aromatic residues, including tyrosine (tyrosine), which is abundantly present in the bio-derived extracellular matrix. The oxidized tyrosine groups are further converted into tyrosyl free radicals and stabilize by forming shared di-tyrosine bonds with nearby tyrosine residues.

FIG. 2 is a schematic diagram showing the process of synthesizing dopamine from oxidized tyrosine according to an exemplary embodiment of the present invention. The Ru/SPS cross-linking system can induce cross-linking very rapidly due to the high absorbance of Ru in the visible light range and its chemical stability in the excited state. Since bio-derived extracellular matrix contains a rich amount of tyrosine transport proteins, this Ru/SPS system can promote the cross-linking of hydrogels more rapidly in a cell-friendly manner. In particular, tyrosine oxidized by Ru reacts with hydroxide ions (HO-) , forming L-3,4-dihydroxyphenylalanine (L-Dopa). This Dopa possesses natural adhesive properties, thereby enhancing the adhesive ability of bio-derived extracellular matrix hydrogels.

Additionally, when the gelatin crosslinking agent is selected from a combination of ruthenium and sodium persulphate, it is preferable that, in consideration of biostability and adhesion ability, the concentration of ruthenium in the bioadhesive should be 0. 1 to 2 mM, and the concentration of sodium peroxymonosulphate is 1 to 20 mM. It is more preferable that the concentration of ruthenium is 0.2 to 1.5 mM and the concentration of sodium peroxymonosulphate is 2 to 15 mM. The inventors added ruthenium and sodium peroxymonosulfate, which are one of the visible light-activated photoinitiators, at final concentrations of 0.5-1 mM and 5-10 mM, respectively, in a 1:10 ratio, and stirred to prepare an extracellular matrix-based adhesive composition. This enabled effective cross-linking through ionization using visible light and the methacrylate polymerisation reaction of methacryloyl-substituted gelatin (GelMA).

The methacryloyl-substituted gelatin may be methacrylated gelatin.

In other words, the methacryloyl-substituted gelatin may be a methacryloyl made from and based on gelatin which is one type of hydrogel. For example, it could be a material created by chemically introducing methacrylate groups into gelatin to enable cross-linking.

The inventors have conducted extensive research to enhance the adhesion of a bioadhesive comprising an extracellular matrix-containing hydrogel and a gelatin hardener, as demonstrated in the following embodiments and experimental examples, it was confirmed that adding metacryloyl-substituted gelatin not only improves viscosity and mechanical strength but also enhances adhesion, leading to the completion of the present invention.

This invention is characterized by including a gelatin cross-linking agent and methacryloyl-substituted gelatin in an extracellular matrix-containing hydrogel, thereby providing an adhesive composition with excellent viscosity and mechanical strength.

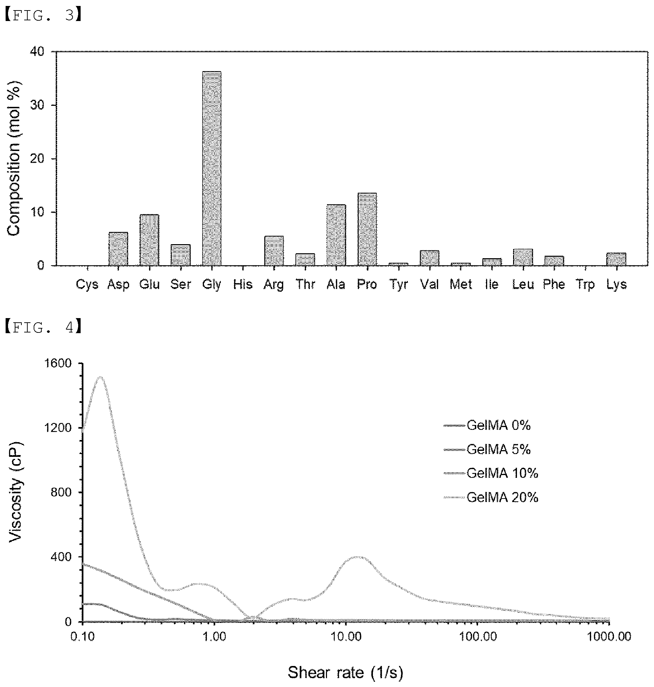

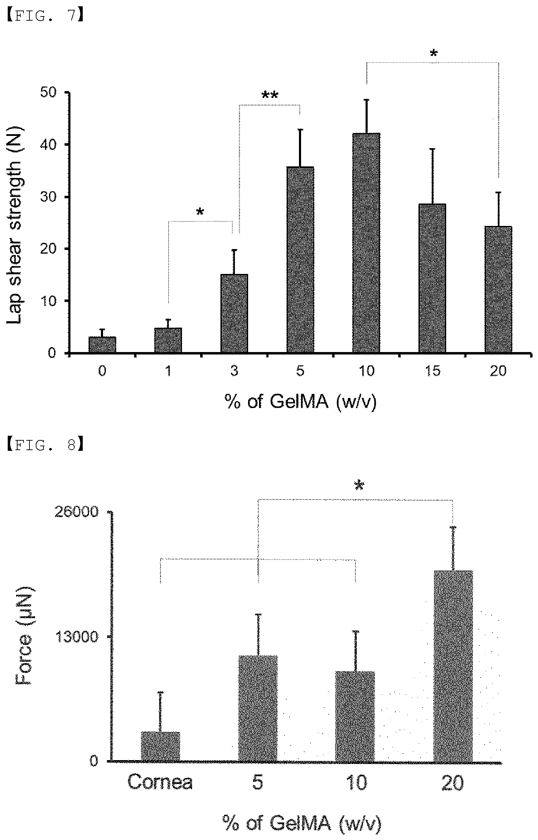

Additionally, as an exemplary embodiment of the present invention, the methacryloyl-substituted gelatin may be included at a concentration of 5 to 15% (w/v) as GelMA (gelatin-methacryloyl), may be included at a concentration of 5 to 10% (w/v), and is preferably included at a concentration of 5% (w/v). As can be seen in the following embodiments and experimental examples, when the methacryloyl-substituted gelatin is included within the above-mentioned range, it not only exhibits excellent viscosity and mechanical strength (see FIGS. 4, 5, and 6) but also demonstrates the best adhesive strength and elasticity (see FIGS. 7 and 8). In other words, when the content of the methacryloyl-substituted gelatin is below the aforementioned range, it has the drawback of insufficient adhesion, viscosity, mechanical strength, and both adhesion and elasticity. On the other hand, when the content exceeds the aforementioned range, it has the drawback of reduced adhesion and excessively high elasticity.

The present invention provides an adhesive composition that not only exhibits excellent biocompatibility but also possesses elasticity similar to that of biological corneal tissue in response to compression stimulation, by incorporating the aforementioned methacryloyl-substituted gelatin in a specific ratio.

As described above, the present invention has elasticity similar to that of biological corneal tissue, making it suitable for use in corneal tissue repair and providing useful applications as an adhesive biomaterial for the healing and/or regeneration of corneal defects and/or damage, such as dry eye syndrome or corneal ulcers.

Accordingly, the extracellular matrix-based adhesive composition according to the present invention can be used for corneal tissue repair. The composition of the present invention is an adhesive tissue repair material, and when composed of corneal matrix-derived extracellular matrix, it is clear and transparent and exhibits a light transmittance of 90% or more. Additionally, it exhibits adhesive properties, maintaining high adhesion even on moist ocular surfaces, and demonstrates high elasticity against shear stresses such as rubbing or blinking. It can be composed of an elastic biopolymer that exhibits elasticity similar to that of biological tissue when subjected to vertical compressive stress.

Furthermore, the adhesive composition according to the present invention can be used as a corneal repair biomaterial useful for healing and inducing regeneration of tissue and reconstructing tissue in cases of corneal damage and deficiency caused by various causes such as Sjögren's syndrome, neurotrophic keratitis, keratoconus, and corneal ulcers. Such corneal tissue repair material compositions exhibit biocompatibility with corneal tissue, are biodegradable, and remain in the corneal tissue for more than 30 days post-implantation, and can be structurally replaced by regenerated autologous corneal tissue.

The method of using the extracellular matrix-based adhesive composition according to an exemplary embodiment of the present invention may comprise: applying the adhesive composition to the lesion site and applying heat or light. For example, when the extracellular matrix-based adhesive composition includes a combination of ruthenium and sodium peroxymonosulphate as a gelatin crosslinking agent, and the adhesive composition is applied to the lesion site and exposed to visible light, specifically blue light with a wavelength of 350-500 nm (preferably 400-450 nm) for 20-120 seconds (preferably 30-100 seconds), a bioadhesive film with excellent adhesion and elasticity can be formed.

When the adhesive composition according to the present invention is actually applied to the surface of the eye and exposed to blue light for 30 seconds to 2 minutes, the adhesive strength (adhesive bond strength), which is the force that resists shear stress, can be secured at 36 to 42 N.

Additionally, when the adhesive composition according to the present invention is subjected to a compressive load that causes it to be compressed to a depth of 100 µm while acting substantially perpendicular to the ocular surface, the compressive stress (compressive stress), which is the resistance force generated in response to the magnitude of the load, is approximately 9,500 to 11,000 µN, thereby imparting characteristics similar to those of biological corneal tissue.

The present invention may be better understood through the following embodiments, which are provided for illustrative purposes and are not intended to limit the scope of protection claimed in the appended claims.

### Manufacturing example: Preparation of corneal-derived decellularized extracellular matrix (Co-dECM) hydrogel

Corneal-derived decellularized extracellular matrix (Co-dECM) hydrogel was prepared as follows. First, the entire cornea excised from pig eyes was washed with PBS buffer solution containing 100 units/ml penicillin and 0.1 mg/ml streptomycin. Next, the epithelium and endothelium were removed from the corneal tissue to obtain the pure corneal stromal layer. The stromal tissue was then placed in a 20 mM ammonium hydroxide solution (NH₄OH; 4.98 N aqueous solution) containing 0.5% Triton X-100 and stirred for approximately 4 hours. The stromal tissue was then washed with distilled water and treated with Tris-HCl (hypotonic Tris hydrochloride; pH 7.4) buffer solution for approximately 24 hours. Subsequently, the substrate tissue was placed in a 10 mM Tris-HCl solution containing 1% (v/v) Triton X-100 and stirred at 37°C for approximately 24 hours to obtain corneal-derived decellularized extracellular matrix (Co-dECM) tissue. Subsequently, the corneal decellularized extracellular matrix (Co-dECM) tissue was sterilized by treating it with a 1% peracetic acid solution in 50% ethanol for approximately 10 hours. After completing the decellularisation process, the corneal-derived decellularized extracellular matrix (Co-dECM) was freeze-dried overnight and ground into a fine powder using liquid nitrogen and a grinding device. 0.2 g of Co-dECM powder was added to 10 mL of acetic acid solution (0.5 M) supplemented with 0.02 g of pepsin, and the mixture was stirred for 3 days to obtain a homogeneous corneal-derived decellularized extracellular matrix hydrogel with a concentration of 2% (w/v). The resulting 2% (w/v) Co-dECM hydrogel was filtered through a 100-micron mesh and stored at 4°C for use in subsequent experiments.

### Exemplary embodiment: Manufacturing of extracellular matrix-based adhesive composition

In the above manufacturing example, 10 N sodium hydroxide solution was added to the 2% (w/v) Co-dECM hydrogel prepared above, and the mixture was stirred to neutralize the pH to 7.0-7.4. The neutralized Co-dECM hydrogel was then heated to 50-56°C and maintained at this temperature for 20-40 minutes to thermally denature it, resulting in the formation of gelatinized Co-dECM hydrogel. Subsequently, the gelatinized Co-dECM hydrogel was slowly cooled to room temperature, and GelMA was added at concentrations of 0, 1, 3, 5, 10, 15, and 20% (w/v) when the temperature reached approximately 37°C, and the mixture was stirred to prepare the biomaterial composition. To this, one of the visible light-activated photoinitiators, ruthenium (Ruthenium), and sodium persulfate (Sodium persulfate) were added at final concentrations of 0.5 mM and 5 mM, respectively, in a 1:10 ratio, and stirred to prepare an extracellular matrix-based adhesive composition. The solvent for both the ruthenium solution and the sodium persulfate solution was DPBS (Dulbecco's phosphate-buffered saline).

### Experimental Example 1: Analysis of amino acid composition of corneal-derived decellularized extracellular matrix (Co-dECM) hydrogel

The protein amino acid composition of the corneal-derived decellularized extracellular matrix (Co-dECM) hydrogel prepared in the above manufacturing example was analyzed using proteomics analysis methods.

The results are shown in FIG. 3. FIG. 3 shows the protein amino acid analysis results of the Co-dECM hydrogel according to an exemplary embodiment of the present invention. As shown in the figure, the major amino acids constituting the proteins in the Co-dECM hydrogel were glycine (Gly, 36%), alanine (Ala, 11.3%), proline (Pro, 13.5%), and glutamic acid (Glu, 9.4%), and it was confirmed that these values are similar to those of collagen composition found in the body.

However, tyrosine (Tyr, 0.39%), a target amino acid component for cross-linking and adhesion induction within the ECM, was found to be present in relatively small amounts.

### Experimental Example 2: Viscosity analysis of extracellular matrix-based adhesive compositions

To analyze the rheological properties of the extracellular matrix-based adhesive composition manufactured in the above embodiment, viscosity was measured using a rheometer.

The results are shown in FIG. 4. FIG. 4 shows the results of viscosity measurements of the extracellular matrix-based adhesive composition according to an embodiment of the present invention as a function of the GelMA addition ratio. The experimental results showed that the composition exhibited shear thinning rheological properties, where viscosity decreased as the rotational shear rate increased under all experimental conditions. Additionally, it was confirmed that the higher the concentration of GelMA mixed into the gelatinized Co-dECM hydrogel, the higher the viscosity.

### Experimental Example 3: Gelation kinetics and mechanical strength (storage modulus) analysis of extracellular matrix-based adhesive compositions

The extracellular matrix-based adhesive composition manufactured in the above embodiment was analyzed using a rheometer while exposing it to blue light (400-500 nm) to analyze the gelation dynamics. When blue light with a wavelength of approximately 400-500 nm is irradiated onto the adhesive composition according to the present invention, the tyrosine residues present within the gelatinized Co-dECM hydrogel are oxidized and converted into tyrosyl free radicals, forming covalent bonds with nearby tyrosine residues to promote hardening.

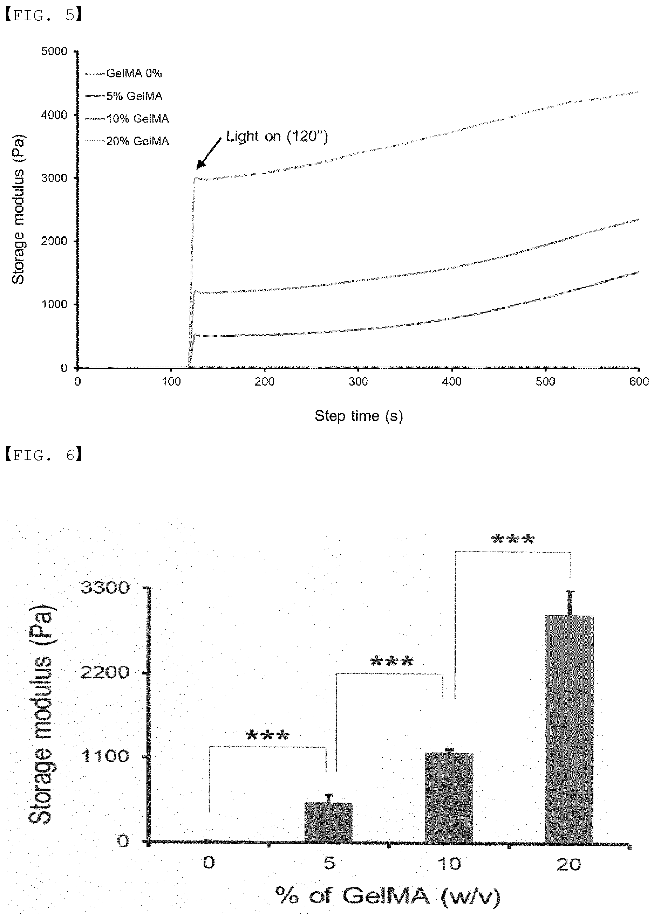

FIG. 5 shows the results of analyzing the gelation kinetics of the extracellular matrix-based adhesive composition according to the present invention, based on the GelMA addition ratio and the visible light irradiation time. As shown in the figure, in all experimental groups, mechanical strength increased sharply immediately after blue light irradiation, confirming that cross-linking was induced simultaneously with blue light irradiation. However, even after continuing blue light irradiation for 10 minutes, the mechanical strength did not increase as sharply as at the initial moment of blue light irradiation.

FIG. 6 shows the results of analyzing the mechanical strength (storage modulus) of gels induced by photopolymerization in an extracellular matrix-based adhesive composition according to an embodiment of the present invention, depending on the GelMA addition ratio. As shown in the figure, the mechanical strength of the gel formed upon blue light exposure increased significantly with an increase in the GelMA mixing ratio. Specifically, the 2% (w/v) Co-dECM hydrogel had a mechanical strength of 4.3 ± 6.62 Pa, while the 2% (w/v) Co-dECM hydrogel mixed with 5% (w/v) GelMA had a mechanical strength of 512. 7 ± 95.96 Pa, while compositions with 10% (w/v) and 20% (w/v) GelMA showed values of 1184.8 ± 36.49 Pa and 2976.8 ± 332.2 Pa, respectively.

### Experimental Example 4: Analysis of adhesive strength (lap shear strength) of extracellular matrix-based adhesive compositions

The adhesive strength of the extracellular matrix-based adhesive composition manufactured in the above embodiment was analyzed by quantifying the shear stress strength using an Instron system.

FIG. 7 shows the results of analyzing the adhesive strength (lap shear strength) of the extracellular matrix-based adhesive composition according to an embodiment of the present invention as a function of the GelMA addition ratio. As shown in the figure, when GelMA was mixed into the gelatinized Co-dECM hydrogel at concentrations of 1, 3, 5, and 10% (w/v), the shear stress strength increased significantly with increasing GelMA concentration. However, when GelMA was mixed at a concentration of 15% (w/v), the shear stress strength decreased, and it decreased further at a concentration of 20% (w/v).

Accordingly, it was found that mixing GelMA at concentrations of 5% to 15% in Co-dECM hydrogel resulted in significantly superior adhesion (36 ± 7 newtons), the highest adhesive strength was observed when GelMA was mixed at approximately 10%, and it was found that mixing GelMA at approximately 5% enables the economical and efficient production of adhesives with excellent adhesive strength.

### Experimental Example 5: Analysis of elasticity of extracellular matrix-based adhesive compositions

The elasticity of the extracellular matrix-based adhesive composition manufactured in the above embodiment was measured in response to compression stimulation.

Specifically, the extracellular matrix-based adhesive composition prepared in the above embodiment was exposed to blue light (400-500 nm) for 2 minutes, and the elasticity of each gelified composition in response to compression stimulation was measured using a compression test with the CellScale MicroTester G2 device and compared with that of actual rat eye tissue.

FIG. 8 shows the results of analyzing elasticity, depending on the GelMA addition ratio, in the extracellular matrix-based adhesive composition according to an embodiment of the present invention. As shown in the figure, when GelMA was mixed into the gelatinized Co-dECM hydrogel at concentrations of 5 and 10% (w/v), the composition exhibited elasticity comparable to that of actual eye tissue when subjected to a compression stimulus of 100 µm in depth. In other words, the compositions containing 5 and 10% (w/v) GelMA were confirmed to possess elasticity capable of withstanding intraocular pressure equivalent to that of actual eye tissue. In comparison, the composition containing GelMA at a concentration of 20% (w/v) exhibited elasticity six times higher than that of corneal tissue.

Accordingly, when the elasticity of adhesive compositions was measured using various mixing ratios of GelMA, it was confirmed once again that when the adhesive composition of the present invention was applied to the cornea, mixing GelMA at 5-10% (w/v) resulted in elasticity similar to that of actual ocular tissue while exhibiting the strongest adhesive strength.

The above description has illustrated and explained the present invention in relation to specific preferred embodiments, but it is clear to those skilled in the art that the present invention may be modified and changed in various ways without departing from the technical features or scope of the present invention as defined by the appended claims.

## Claims

1. An extracellular matrix-based adhesive composition comprising an extracellular matrix-containing hydrogel, a gelatin crosslinking agent, and a methacryloyl-substituted gelatin.

2. The extracellular matrix-based adhesive composition of claim 1, **characterized in that** the methacryloyl-substituted gelatin is included at a concentration of 5 to 15% (w/v) as GelMA (gelatin-methacryloyl).

3. The extracellular matrix-based adhesive composition of claim 2, **characterized in that** the methacryloyl-substituted gelatin is included at a concentration of 5 to 10% (w/v) as GelMA.

4. The extracellular matrix-based adhesive composition of claim 3, **characterized in that** the methacryloyl-substituted gelatin is included at a concentration of 5% (w/v) as GelMA.

5. The extracellular matrix-based adhesive composition of claim 3, **characterized in that** the extracellular matrix-containing hydrogel is a gelatinized form of extracellular matrix derived from corneal stroma.

6. The extracellular matrix-based adhesive composition of claim 5, **characterized in that** the gelatin crosslinking agent is a photoinitiator comprising of combination of ruthenium and sulphuric acid.

7. The extracellular matrix-based adhesive composition of any one claim of 1 to 6, **characterized in that** the composition is for corneal tissue repair.
